# EUROPEAN PATENT APPLICATION

(11) **EP 4 427 770 A1**
(43) Date of publication of application: **11.09.2024**
(21) Application number: 23895543.9
(22) Date of filing: 07.03.2023
(51) Int. Cl.: A61M 5/142

(54) **PATCH INFUSION PUMP AND INFUSION METHOD**

(30) Priority: 18.01.2023 CN 202310056539
(71) Applicant: Wuxi Biohermes Bio & Medical Technology Co., Ltd., Wuxi, Jiangsu 214092 (CN); Wuxi Kas Bio & Medical Technology Co., Ltd., Wuxi, Jiangsu 214135 (CN)
(72) Inventor: ZHANG, Peng, Wuxi, Jiangsu 214092 (CN); HAN, Yukai, Wuxi, Jiangsu 214092 (CN); YAN, Peng, Wuxi, Jiangsu 214092 (CN); WANG, Xiang, Wuxi, Jiangsu 214092 (CN); LIU, Yan, Wuxi, Jiangsu 214092 (CN)
(74) Representative: Regimbeau
(86) International application number: PCT/CN2023/079970
(87) International publication number: WO 2024/152417

(57) **Abstract**

Provided are a patch infusion pump and an infusion method. The patch infusion pump includes an infusion tube configured to deliver a liquid medication; and an infusion assembly including multiple valve mechanisms located at a side of the infusion tube. Each valve mechanism includes an abutting valve, a memory metal member, and a reset elastic member, the memory metal member is connected to the abutting valve, the abutting valve is configured to abut against the infusion tube, the memory metal member contracts when being energized and drives the abutting valve to move away from the infusion tube, and the reset elastic member is configured to reset the abutting valve to a position for abutting against the infusion tube after the memory metal member is de-energized. Memory metal members of the multiple valve mechanisms of the patch infusion pump are energized and de-energized according to a preset sequence for operation to enable deformation of the infusion tube and successive generation of a negative pressure and a positive pressure, the infusion tube is configured to suck the liquid medication from a liquid reservoir under the negative pressure and pump the liquid medication to a medication output mechanism under the positive pressure.

## Description

This application claims priority to Chinese Patent Application No. 202310056539.8 filed with the China National Intellectual Property Administration (CNIPA) on Jan. 18, 2023, the disclosure of which is incorporated herein by reference in its entirety.

### TECHNICAL FIELD

The present application relates to the technical field of medical devices, for example, a patch infusion pump and an infusion method.

### BACKGROUND

Diabetes has become a global public health problem since the diabetes impacts on human health. As an effective means of blood glucose management for patients with diabetes, an insulin pump is a micro-precision and continuously controllable subcutaneous insulin injection tool, which can better simulate a physiological insulin secretion pattern. Existing insulin pumps mainly include two types: line pumps and patch pumps. A line pump consists of a pump body, a medicine reservoir, and an infusion set with a pipeline. The infusion set is attached to the skin of a patient and connected to the pump body through an external pipeline, and a plunger of the infusion set is driven through a reduction motor of the pump body so that insulin can flow in the infusion set. The line pump has been very mature. However, the patient needs to pay close attention while using the line pump due to the external pipeline and the heavy pump body, and the wearing experience is very bad for the patient.

A patch pump is also referred to as a catheterless pump. Compared with the line pump, the patch pump is relatively small in volume and relatively light in weight. Therefore, the whole patch pump can be directly patched with the skin of a patient for infusion treatment, which is convenient for the patient to use. Moreover, the wearing of the patch pump hardly affects daily activities of the patient, and the user experience of the patient is improved to a certain extent.

However, since the patch pump needs to be worn for a long time, patients put higher requirements on the volume and weight of the patch pump. An infusion mechanism is an important component of the patch pump, and the structure of the infusion mechanism restrains the volume and weight of the patch pump from being further reduced. An existing infusion mechanism adopts one of the following ways to drive a reduction gear to push a plunger: using a miniature reduction motor with smaller volume than that in the infusion scheme of the line pump or using a shape-memory alloy (SMA). Both the two above ways have limited contributions to reducing the volume and weight of the patch pump and cannot meet the requirements of users on the volume and weight of the patch pump.

### SUMMARY

The present application provides a patch infusion pump and an infusion method so that the requirements of users for a small volume and a light weight of the patch infusion pump can be satisfied, user experience for long time wearing can be improved, and the reliability and accuracy with which a liquid medication is infused are also improved.

Embodiments of the present application provide a patch infusion pump including the following: an infusion tube configured to deliver a liquid medication; and an infusion assembly including multiple valve mechanisms located at a side of the infusion tube and arranged along a flow direction of the liquid medication in the infusion tube with spacings. Each valve mechanism includes an abutting valve, a memory metal member, and a reset elastic member, the abutting valve is configured to abut against the infusion tube to block a flow of the liquid medication in the infusion tube, the memory metal member contracts when being energized and drives the abutting valve to move away from the infusion tube, and the reset elastic member is configured to reset the abutting valve to a position for abutting against the infusion tube after the memory metal member is de-energized. Memory metal members of the multiple valve mechanisms are energized and de-energized according to a preset sequence to enable deformation of the infusion tube and successive generation of a negative pressure and a positive pressure in the infusion tube, the infusion tube is configured to suck the liquid medication from a liquid reservoir under the negative pressure in the infusion tube and pump the liquid medication to a medication output mechanism under the positive pressure in the infusion tube.

As an optional embodiment, the infusion assembly further includes a base. The base includes multiple channel plates, a valve channel is formed between two adjacent channel plates of the multiple channel plates, and each abutting valve is movably disposed in a respective valve channel.

As an optional embodiment, the base further includes a channel baffle, the channel baffle is disposed on one side of the multiple channel plates, two adjacent channel plates are spaced apart, and the infusion tube is located between the channel baffle and the multiple channel plates.

As an optional embodiment, the base further includes a limiting plate, the limiting plate is disposed on the other side of the multiple channel plates, and multiple limiting holes are formed on the limiting plate; and each abutting valve includes a valve abutting head and a valve guide rod that are connected to each other, the valve abutting head is configured to abut against the infusion tube, and the valve guide rod penetrates through one limiting hole of the multiple limiting holes.

As an optional embodiment, each abutting valve further includes a valve connecting seat connected between the valve abutting head and the valve guide rod; and the reset elastic member is a coil spring sleeved on the valve guide rod, one end of the coil spring abuts against the valve connecting seat in a limiting manner, and the other end of the coil spring abuts against the limiting plate in a limiting manner.

As an optional embodiment, the infusion assembly further includes an infusion cover plate fixedly disposed on the base; and the memory metal member includes a positive electrode elastic sheet, a memory metal wire, and a negative electrode elastic sheet that are connected successively, one of the positive electrode elastic sheet and the negative electrode elastic sheet is connected to the abutting valve, and the other of the positive electrode elastic sheet and the negative electrode elastic sheet is connected to the infusion cover plate.

As an optional embodiment, the infusion assembly further includes a guide post provided with limiting guide slots along the circumferential direction of the guide post; and the infusion cover plate and the abutting valve are disposed up and down, the guide post is disposed on a side of the infusion cover plate and the abutting valve, the memory metal wire is disposed around the guide post and positioned in a respective one of the limiting guide slots.

As an optional embodiment, the infusion assembly further includes a ground elastic sheet, the patch infusion pump further includes a circuit board, the positive electrode elastic sheet is electrically connected to the circuit board, and the negative electrode elastic sheet is electrically connected to the circuit board through the ground elastic sheet.

As an optional embodiment, the patch infusion pump further includes a housing, the liquid reservoir, a power supply mechanism, and the medication output mechanism. A mounting cavity is formed in the housing, the infusion assembly, the liquid reservoir, and the power supply mechanism are all disposed in the mounting cavity, a liquid inlet end of the infusion tube communicates with the liquid reservoir, a liquid outlet end of the infusion tube communicates with the medication output mechanism, and the power supply mechanism is configured to supply power to the patch infusion pump.

Embodiments of the present application further provide an infusion method applied in the preceding patch infusion pump. An infusion assembly of the patch infusion pump includes an infusion tube and multiple valve mechanisms, and the multiple valve mechanisms include a first valve mechanism located most upstream, a third valve mechanism located most downstream, and a second valve mechanism located between the first valve mechanism and the third valve mechanism along the flow direction of the liquid medication in the infusion tube. The infusion method includes that the infusion assembly performs one infusion operation according to the following sequence: a memory metal member of the first valve mechanism is energized, a memory metal member of the second valve mechanism is energized, the memory metal member of the first valve mechanism is de-energized, a memory metal member of the third valve mechanism is energized, the memory metal member of the second valve mechanism is de-energized, and the memory metal member of the third valve mechanism is de-energized.

### BRIEF DESCRIPTION OF DRAWINGS

FIG. 1 is an exploded view of a patch infusion pump according to an embodiment of the present application;
FIG. 2 is a top view of some structures of a patch infusion pump according to an embodiment of the present application;
FIG. 3 is an exploded view of some structures of a patch infusion pump according to an embodiment of the present application;
FIG. 4 is a sectional view of a patch infusion pump according to an embodiment of the present application;
FIG. 5 is a schematic view of an abutting valve of a patch infusion pump according to an embodiment of the present application;
FIG. 6 is a schematic view of a memory metal member of a patch infusion pump according to an embodiment of the present application; and
FIG. 7 is a schematic view of a ground elastic sheet of a patch infusion pump according to an embodiment of the present application.

### Reference list:

- 1: infusion assembly
- 9: valve mechanism
- 91: first valve mechanism
- 92: second valve mechanism
- 93: third valve mechanism
- 110: abutting valve
- 111: valve abutting head
- 112: valve guide rod
- 113: valve connecting seat
- 114: second protruding post
- 120: memory metal member
- 121: positive electrode elastic sheet
- 1211: first insertion hole
- 122: memory metal wire
- 123: negative electrode elastic sheet
- 1231: second insertion hole
- 130: reset elastic member
- 140: base
- 141: base plate
- 142: channel plate
- 143: channel baffle
- 144: limiting plate
- 1441: limiting hole
- 150: infusion cover plate
- 151: first protruding post
- 160: guide post
- 161: limiting guide slot
- 170: ground elastic sheet
- 171: common ground base plate
- 172: common ground elastic pin
- 2: infusion tube
- 3: housing
- 4: liquid reservoir
- 5: circuit board
- 6: power supply mechanism
- 7: lance needle driver
- 8: lance needle
- 10: medication output mechanism

### DETAILED DESCRIPTION

Technical schemes of the present application are described below in conjunction with the drawings. Apparently, the embodiments described herein are part of embodiments of the present application. Based on the embodiments of the present application, all other embodiments obtained by those of ordinary skill in the art without creative work are within the scope of the present application.

In the description of the present application, it is to be noted that orientations or position relations indicated by terms such as "center", "upper", "lower", "left", "right", "vertical", "horizontal", "in", and "out" are based on the drawings. These orientations or position relations are intended only to facilitate and simplify the description of the present application and not to indicate or imply that a device or element referred to must have such particular orientations or must be configured or operated in such particular orientations. Thus, these orientations or position relations are not to be construed as limiting the present application. Moreover, terms such as "first" and "second" are used only for the purpose of description and are not to be construed as indicating or implying relative importance. Terms such as "first position" and "second position" refer to two different positions.

In the description of the present application, it is to be noted that terms "mounted", "joined", and "connected" are to be understood in a broad sense unless otherwise expressly specified and limited. For example, the term "connected" may refer to "securely connected" or "detachably connected", may refer to "mechanically connected" or "electrically connected", or may refer to "connected directly", "connected indirectly through an intermediary", or "connected inside two components". For those of ordinary skill in the art, meanings of the preceding terms in the present application may be understood based on situations.

The present application provides a patch infusion pump. The patch infusion pump is applicable to a body of a patient and pumps insulin into the body of the patient. Of course, in addition to the insulin, the patch infusion pump may be configured to pump other liquid medications into the body of the patient.

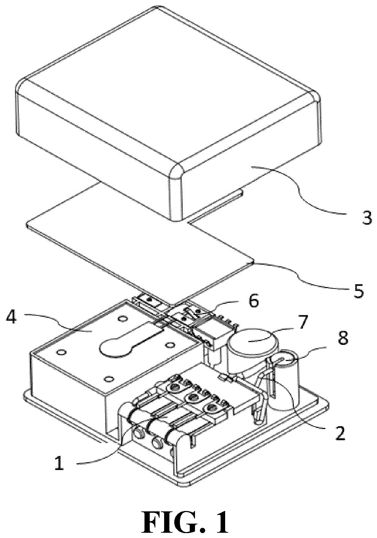

As shown in FIGS. 1 to 4, the patch infusion pump includes an infusion tube 2 and an infusion assembly 1. The infusion tube 2 is configured to deliver a liquid medication such as the insulin. The infusion assembly 1 includes multiple valve mechanisms 9 located at a side of the infusion tube 2 and arranged along a flow direction of the liquid medication in the infusion tube 2 with spacings. It is to be noted that the multiple valve mechanisms 9 may be located on the same side of the infusion tube 2 or may be located on different sides of the infusion tube 2. Each valve mechanism 9 includes an abutting valve 110, a memory metal member 120, and a reset elastic member 130. The abutting valve 110 is configured to abut against the infusion tube 2 to block a flow of the liquid medication in the infusion tube 2. The memory metal member 120 is a long strip made of a memory metal, and the memory metal is a special metal material that is subjected to plastic deformation within a certain temperature range and then can restore to its original macro shape within another temperature range. The memory metal member 120 contracts when being energized and drives the abutting valve 110 to move away from the infusion tube 2. After the memory metal member 120 is de-energized, the reset elastic member 130 is configured to reset the abutting valve 110 to a position for abutting against the infusion tube 2. Memory metal members 120 of the multiple valve mechanisms 9 are energized and de-energized according to a preset sequence for operation to enable deformation of the infusion tube 2 and successive generation of a negative pressure and a positive pressure inside the infusion tube 2. The infusion tube 2 sucks the liquid medication from a liquid reservoir 4 under the negative pressure in the infusion tube 2, and the infusion tube 2 pumps the liquid medication to a medication output mechanism 10 under the positive pressure in the infusion tube 2.

Along the flow direction of the liquid medication in the infusion tube 2, the multiple valve mechanisms 9 include a first valve mechanism 91 located most upstream, a third valve mechanism 93 located most downstream, and a second valve mechanism 92 located in the middle. When the abutting valve 110 of the third valve mechanism 93 located most downstream is in a state of abutting against the infusion tube 2, the memory metal member 120 of the first valve mechanism 91 and the memory metal member 120 of the second valve mechanism 92 that are located upstream of the third valve mechanism 93 are sequentially energized, and the corresponding abutting valve 110 moves away from the infusion tube 2 so that the negative pressure is formed in the infusion tube 2, and the infusion tube 2 sucks the liquid medication to the next valve mechanism under the negative pressure. Then, the memory metal member 120 of the first valve mechanism 91 located most upstream is de-energized, and the corresponding abutting valve 110 abuts against the infusion tube 2 again so that the infusion tube 2 stops sucking the liquid medication. Next, the memory metal member 120 of the third valve mechanism 93 located most downstream is energized, and the corresponding abutting valve 110 moves away from the infusion tube 2 so that the infusion tube 2 communicates with the medication output mechanism 10. Finally, when the abutting valve 110 of the first valve mechanism 91 located most upstream is in the state of abutting against the infusion tube 2 again, the memory metal member 120 of the second valve mechanism 92 and the memory metal member 120 of the third valve mechanism 93 that are located downstream of the first valve mechanism 91 are sequentially de-energized, and the corresponding abutting valve 110 abuts against the infusion tube 2 again so that the positive pressure is formed in the infusion tube 2, and the infusion tube 2 pumps the liquid medication out under the positive pressure.

The patch infusion pump provided in the present application is small in volume and light in weight, which not only meets requirements of the patient and improves the wearing experience of the patient but also simplifies and facilitates the assembly of the patch infusion pump. Additionally, the multiple valve mechanisms of the patch infusion pump mate with each other so that higher infusion accuracy is obtained, and the multiple valve mechanisms block the infusion tube 2 when not in action so that the liquid medication is reliably isolated from the human body, achieving higher safety.

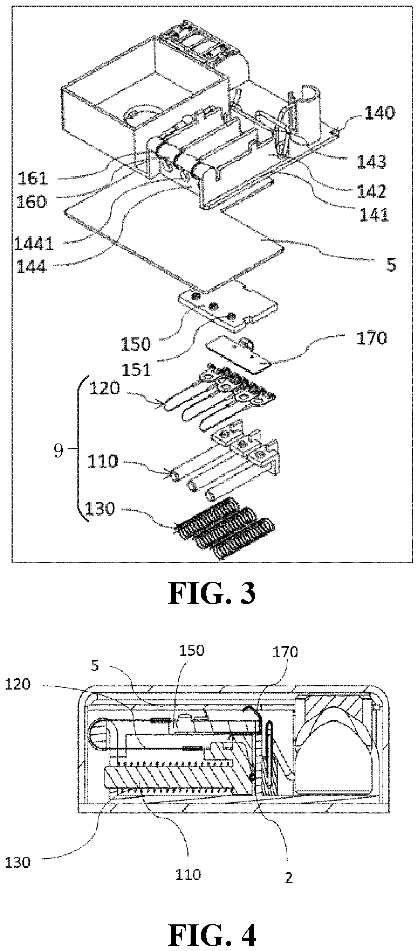

In some embodiments, with continued reference to FIG. 3, the infusion assembly 1 further includes a base 140, and the base 140 is used for mounting the infusion tube 2 and the multiple valve mechanisms 9. The base 140 includes a base plate 141 and multiple channel plates 142 arranged on the base plate 141, the multiple channel plates 142 are arranged in parallel, each channel plate 142 is disposed along a direction perpendicular to the infusion tube 2, a valve channel is formed between two adjacent channel plates 142, the number of valve channels is the same as the number of valve mechanisms, each abutting valve 110 is movably disposed in a respective valve channel, and the valve channel is configured to guide the movement of the abutting valve 110.

In an embodiment, the patch infusion pump is provided with three valve mechanisms 9, and correspondingly, four channel plates 142 are arranged on the base 140. Three valve channels are formed between the four channel plates 142. In other embodiments, four, five, or more valve mechanisms 9 may be arranged as required, and correspondingly, the number of channel plates 142 may be increased to form more valve channels.

To limit the position of the infusion tube 2 so that the valve mechanism 9 can accurately abut against or be disengaged from the infusion tube 2, with continued reference to FIG. 3, the base 140 further includes a channel baffle 143, the channel baffle 143 is disposed on one side of the multiple channel plates 142, two adjacent channel plates 142 are spaced apart, and a space for accommodating the infusion tube 2 is formed. The infusion tube 2 is located between the channel baffle 143 and the multiple channel plates 142. In some embodiments, the thickness of a tube wall of the infusion tube 2 is less than 0.5 mm.

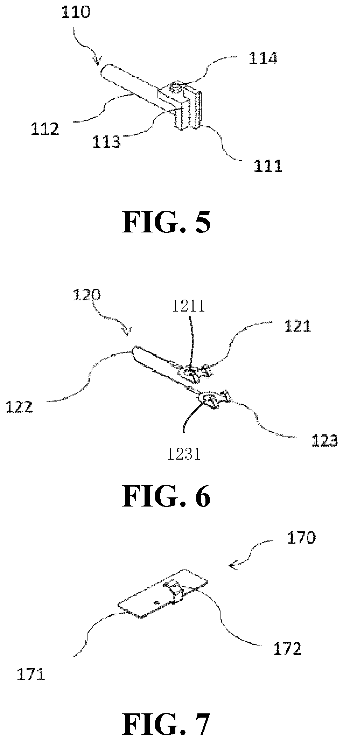

To further improve the movement accuracy of the abutting valve 110, with continued reference to FIG. 3, the base 140 further includes a limiting plate 144, the limiting plate 144 is disposed on the other side of the multiple channel plates 142, multiple limiting holes 1441 are formed on the limiting plate 144, and the number of limiting holes 1441 is the same as the number of valve mechanisms 9. As shown in FIG. 5, the abutting valve 110 includes a valve abutting head 111 and a valve guide rod 112 that are connected to each other, the valve abutting head 111 is configured to abut against the infusion tube 2, the valve guide rod 112 penetrates through a limiting hole 1441, and the valve guide rod 112 is capable of moving in the limiting hole 1441, thereby improving the movement accuracy of the valve mechanism 9. In an embodiment, the number of valve guide rods 112 is three, and correspondingly, the number of limiting holes 1441 on the limiting plate 144 is also three. In an embodiment, the valve guide rod 112 is a cylindrical rod, and the limiting hole 1441 is a circular hole. Of course, cross-sections of the valve guide rod 112 and the limiting hole 1441 may also be in other shapes.

In some embodiments, with continued reference to FIG. 5, the abutting valve 110 further includes a valve connecting seat 113 connected between the valve abutting head 111 and the valve guide rod 112. The reset elastic member 130 is a coil spring sleeved on the valve guide rod 112, one end of the coil spring abuts against the valve connecting seat 113 in a limiting manner, and the other end of the coil spring abuts against the limiting plate 144 in a limiting manner. In this manner, the coil spring can be fixed and limited. In an embodiment, the valve connecting seat 113 is an L-shaped plate, and one end of the valve guide rod 112 is connected to an inner wall surface of a vertical plate of the valve connecting seat 113; and the abutting valve 110 is an L-shaped plate, and the abutting valve 110 is connected to an outer wall surface of the valve connecting seat 113.

With continued reference to FIG. 4, one end of the memory metal member 120 is fixed, which means that one end of the memory metal member 120 is fixed to another mechanism so that the end of the memory metal member 120 cannot be moved. The other end of the memory metal member 120 is connected to the abutting valve 110, where the "connected" here may refer to directly and securely connected or may refer to abutting through a rotatable cam. When the memory metal member 120 is directly connected to the abutting valve 110, the memory metal member 120 contracts to directly pull the abutting valve 110 to move. When the memory metal member 120 rotatably abuts against the abutting valve 110 through the cam, the memory metal member 120 deforms to drive the cam to rotate, and different positions of the cam abut against the abutting valve 110 to enable the abutting valve 110 to move.

In some embodiments, as shown in FIG. 6, the memory metal member 120 includes a positive electrode elastic sheet 121, a memory metal wire 122, and a negative electrode elastic sheet 123 that are connected successively. The memory metal wire 122 is a wire structure made of the memory metal, and both the positive electrode elastic sheet 121 and the negative electrode elastic sheet 123 are made of a conductive material. To fix one end of the memory metal member 120, with continued reference to FIGS. 3 and 4, the infusion assembly 1 further includes an infusion cover plate 150, the infusion cover plate 150 is fixedly disposed on the base 140, for example, may be fixedly connected to the channel baffle 143, one of the positive electrode elastic sheet 121 and the negative electrode elastic sheet 123 is connected to the abutting valve 110, and the other of the positive electrode elastic sheet 121 and the negative electrode elastic sheet 123 is connected to the infusion cover plate 150.

In an embodiment, the positive electrode elastic sheet 121 and the negative electrode elastic sheet 123 have the same structure, the positive electrode elastic sheet 121 is connected to the infusion cover plate 150, and the negative electrode elastic sheet 123 is connected to the valve connecting seat 113 of the abutting valve 110. To quickly connect the positive electrode elastic sheet 121 to the infusion cover plate 150, the positive electrode elastic sheet 121 is provided with a first insertion hole 1211, the infusion cover plate 150 is provided with a first protruding post 151, and the first protruding post 151 can be inserted into the first insertion hole 1211. To quickly connect the negative electrode elastic sheet 123 to the valve connecting seat 113, the negative electrode elastic sheet 123 is provided with a second insertion hole 1231, the valve connecting seat 113 is provided with a second protruding post 114, and the second protruding post 114 can be inserted into the second insertion hole 1231.

To reduce an area occupied by the patch infusion pump when being applied to the patient on the premise of maintaining a movable distance of the abutting valve 110, with continued reference to FIGS. 3 and 4, the infusion assembly 1 further includes a guide post 160, the guide post 160 is fixed above the channel plates 142 and located directly above the limiting plate 144, and the guide post 160 is provided with limiting guide slots 161 along the circumferential direction of the guide post 160. The infusion cover plate 150 and the abutting valve 110 are disposed up and down, the guide post 160 is disposed on a side of the infusion cover plate 150 and the abutting valve 110, the memory metal wire 122 is disposed around the guide post 160 and positioned in a respective limiting guide slot 161. In this manner, the memory metal wire 122 is folded into two parts, one part is located on the upper side of the guide post 160, and the other part is located on the lower side of the guide post 160 so that a transverse dimension of the patch infusion pump can be reduced, and the area occupied by the patch infusion pump when being applied to the patient is reduced. In some embodiments, the guide post 160 is semi-cylindrical, and the limiting guide slots 161 are disposed on a semi-cylindrical surface of the guide post 160.

To energize and de-energize the memory metal body, with continued reference to FIGS. 3 and 4, the infusion assembly 1 further includes a ground elastic sheet 170, the patch infusion pump further includes a circuit board 5 which is a printed circuit board (PCB) and is disposed above the infusion cover plate 150, the positive electrode elastic sheet 121 is electrically connected to the circuit board 5 directly, a part of the ground elastic sheet 170 is located between the abutting valve 110 and the infusion cover plate 150, and the other part of the ground elastic sheet 170 is around the infusion cover plate 150, located above the infusion cover plate 150, and electrically connected to the circuit board 5, that is, the negative electrode elastic sheet 123 is electrically connected to the circuit board 5 through the ground elastic sheet 170.

In some embodiments, as shown in FIG. 7, the ground elastic sheet 170 includes a common ground base plate 171 and a common ground elastic pin 172, the common ground base plate 171 is a rectangular plate and located between the abutting valve 110 and the infusion cover plate 150, the common ground elastic pin 172 is an elastic curved plate, and the elastic curved plate can be compressed and deformed so that the common ground base plate 171 can always be electrically connected to the circuit board 5 when the abutting valve 110 is moved.

The circuit board 5 may be a centralized or distributed controller. For example, the circuit board 5 may be one separate single-chip microcomputer or be composed of multiple distributed single-chip microcomputers. The single-chip microcomputer may run control programs to control the multiple valve mechanisms 9 to implement energization and de-energization functions according to a preset sequence.

To protect the valve mechanisms 9 and other components disposed on the base 140, with continued reference to FIG. 1, the patch infusion pump further includes a housing 3, the housing 3 is snap-fitted on the base plate 141 of the base 140, a mounting cavity is formed between the housing 3 and the base plate 141, and components such as the valve mechanisms 9 are all disposed in the mounting cavity.

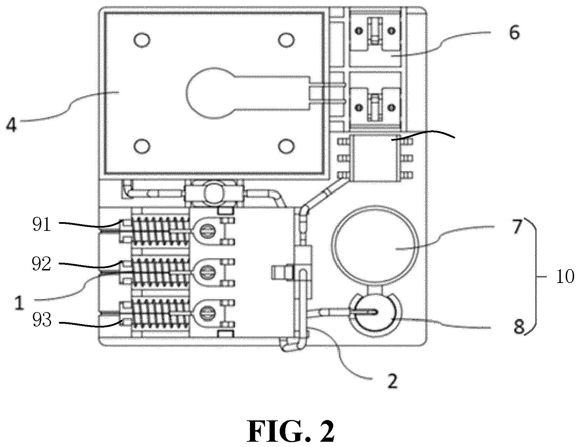

With continued reference to FIGS. 1 and 2, the patch infusion pump further includes the liquid reservoir 4 and the medication output mechanism, the liquid reservoir 4 is disposed in the mounting cavity and fixed to the base plate 141 of the base 140, the liquid reservoir 4 has a liquid storage cavity for storing the liquid medication, and the liquid reservoir 4 communicates with a liquid inlet end of the infusion tube 2 so that the liquid medication in the liquid storage cavity can flow into the infusion tube. The medication output mechanism is configured to output the liquid medication. In some embodiments, the medication output mechanism includes a lance needle driver 7 and a lance needle 8, the lance needle driver 7 is disposed in the mounting cavity and fixed to the base plate 141 of the base 140, the lance needle 8 is connected to an outlet end of the infusion tube 2, and the lance needle driver 7 is configured to drive the lance needle 8 to move so that the lance needle 8 is lanced to or removed from the body of the patient.

In an embodiment, the lance needle driver 7 is rotatably connected to the base plate 141, the lance needle driver 7 is provided with a spiral slot along the circumferential direction of the lance needle driver 7, the lance needle 8 is provided with a protruding block, and the protruding block is disposed in the spiral slot. The lance needle driver 7 is driven to rotate so that the protruding block can be driven to be raised and lowered in a vertical direction, thereby moving the lance needle 8 up and down. The rotation of the lance needle driver 7 may be driven manually or driven by a power member such as a motor.

With continued reference to FIGS. 1 and 2, the patch infusion pump further includes a power supply mechanism 6, the power supply mechanism 6 is disposed in the mounting cavity and fixedly connected to the base plate 141, and the power supply mechanism 6 is configured to supply power to electrical components of the patch infusion pump. The electrical components may be the lance needle driver 7, the circuit board 5, and the like. In some embodiments, the power supply mechanism 6 is a button cell, for example, an LR44 button cell, and three LR44 button cells may be used as required. In some embodiments, the power supply mechanism 6 is a battery pack such as a lithium-ion battery pack.

The working principle of the patch infusion pump is described below.

When the patch infusion pump needs to perform infusion, the memory metal member 120 of the valve mechanism 9 is energized and contracts to pull the abutting valve 110 to move away from the infusion tube 2. In this process, the coil spring is compressed, and the infusion tube 2 previously deformed due to the squeeze of the abutting valve 110 restores its shape to generate the negative pressure and suck the medication. When the memory metal member 120 is de-energized, the abutting valve 110 moves towards the infusion tube 2 under the action of the coil spring, the abutting valve 110 squeezes the infusion tube 2 again in collaboration with the channel baffle 143, and the infusion tube 2 decreases in volume to generate the positive pressure and pump the medication out. The multiple valve mechanisms of the patch infusion pump collaborate by energizing and de-energizing according to a particular timing so that the medication is reliably and accurately infused. Moreover, in a non-infusion state, the abutting valve 110 always abuts against the infusion tube 2 under the action of the coil spring to ensure that the medication is isolated from the human body, thereby greatly improving safety.

The present application further provides an infusion method. The infusion method uses the preceding patch infusion pump.

For ease of description, along the flow direction of the liquid medication in the infusion tube 2, the valve mechanism 9 located most upstream among multiple valve mechanisms 9 is referred to as a first valve mechanism 91, the valve mechanism 9 located most downstream among the multiple valve mechanisms 9 is referred to as a third valve mechanism 93, and the valve mechanism 9 located between the first valve mechanism 91 and the third valve mechanism 93 is referred to as a second valve mechanism 92. One, two, or more second valve mechanisms 92 may be provided as required.

With only one second valve mechanism 92 as an example, the infusion method includes the steps below.

The infusion assembly 1 may perform one infusion operation according to the following sequence: the memory metal member 120 of the first valve mechanism 91 is energized (the liquid medication flows to the second valve mechanism 92), the memory metal member 120 of the second valve mechanism 92 is energized (the liquid medication flows to the third valve mechanism 93), the memory metal member 120 of the first valve mechanism 91 is de-energized (the liquid inlet end of the infusion tube is blocked), the memory metal member 120 of the third valve mechanism 93 is energized (the liquid outlet end of the infusion tube is opened), the memory metal member 120 of the second valve mechanism 92 is de-energized (the liquid medication flows out of the second valve mechanism 92), and the memory metal member 120 of the third valve mechanism 93 is de-energized (the liquid medication flows out of the third valve mechanism 93).

It is to be noted that these steps need to be performed at time intervals, so as to provide sufficient time for the flow of the liquid medication.

When two or more second valve mechanisms 92 are provided, as long as the operation step about the second valve mechanism 92 is involved, the operation step is repeated by multiple second valve mechanisms 92 successively from upstream to downstream along the flow direction of the liquid medication in the infusion tube 2. For example, the operation step of "energizing the memory metal member 120 of the second valve mechanism 92" may be energizing the memory metal member 120 of a second valve mechanism 92 located most upstream, then energizing the memory metal member 120 of a next adjacent second valve mechanism 92, ... and finally energizing the memory metal member 120 of a second valve mechanism 92 located most downstream along the flow direction of the liquid medication in the infusion tube 2.

Of course, the multiple second valve mechanisms 92 may simultaneously repeat the operation step. For example, the operation step of "energizing the memory metal member 120 of the second valve mechanism 92" may be simultaneously energizing the memory metal members 120 of the multiple second valve mechanisms 92. It is to be noted that in the process of the circuit board 5 controlling the valve mechanisms 9, it needs to be ensured that at least one valve mechanism 9 is in a state of blocking the infusion tube 2 so that the safety of the patient and the reliability of the patch infusion pump can be ensured.

According to the preceding infusion method, the liquid medication can gradually flow towards the medication output mechanism 10 under the negative pressure, and the multiple valve mechanisms 9 collaborate by energizing and de-energizing according to a particular timing so that the medication can be reliably and accurately infused.

## Claims

1. A patch infusion pump, comprising:
an infusion tube (2) configured to deliver a liquid medication; and
an infusion assembly (1) comprising a plurality of valve mechanisms (9) located at a side of the infusion tube (2) and arranged along a flow direction of the liquid medication with spacings, wherein each of the plurality of valve mechanisms (9) comprises an abutting valve (110), a memory metal member (120), and a reset elastic member (130), wherein
the abutting valve (110) is configured to abut against the infusion tube (2) to block a flow of the liquid medication in the infusion tube (2),
the memory metal member (120) contracts when being energized and drives the abutting valve (110) to move away from the infusion tube (2), and
the reset elastic member (130) is configured to reset the abutting valve (110) to a position for abutting against the infusion tube (2) after the memory metal member (120) is de-energized; wherein
wherein memory metal members (120) of the plurality of valve mechanisms are energized and de-energized according to a preset sequence to enable deformation of the infusion tube (2) and successive generation of a negative pressure and a positive pressure in the infusion tube (2), the infusion tube (2) is configured to suck the liquid medication from a liquid reservoir (4) under the negative pressure in the infusion tube (2) and pump the liquid medication to a medication output mechanism (10) under the positive pressure in the infusion tube (2).

2. The patch infusion pump of claim 1, wherein the infusion assembly (1) further comprises a base (140), the base (140) comprises a plurality of channel plates (142), a valve channel is formed between two adjacent channel plates (142) of the plurality of channel plates (142), and each abutting valve (110) is movably disposed in a respective valve channel.

3. The patch infusion pump of claim 2, wherein the base (140) further comprises a channel baffle (143), the channel baffle (143) is disposed on one side of the plurality of channel plates (142), two adjacent channel plates (142) of the plurality of channel plates (142) are spaced apart, and the infusion tube (2) is located between the channel baffle (143) and the plurality of channel plates (142).

4. The patch infusion pump of claim 2, wherein
the base (140) further comprises a limiting plate (144), the limiting plate (144) is disposed on the other side of the plurality of channel plates (142), and a plurality of limiting holes (1441) are formed on the limiting plate (144); and
each abutment valve (110) comprises a valve abutting head (111) and a valve guide rod (112) that are connected to each other, wherein the valve abutting head (111) is configured to abut against the infusion tube (2), and the valve guide rod (112) penetrates through one limiting hole (1441) of the plurality of limiting holes (1441).

5. The patch infusion pump of claim 4, wherein
each abutting valve (110) further comprises a valve connecting seat (113) connected between the valve abutting head (111) and the valve guide rod (112); and
the reset elastic member (130) is a coil spring sleeved on the valve guide rod (112), one end of the coil spring abuts against the valve connecting seat (113) in a limiting manner, and the other end of the coil spring abuts against the limiting plate (144) in a limiting manner.

6. The patch infusion pump of claim 2, wherein
the infusion assembly (1) further comprises an infusion cover plate (150) fixedly disposed on the base (140); and
the memory metal member (120) comprises a positive electrode elastic sheet (121), a memory metal wire (122), and a negative electrode elastic sheet (123) that are connected successively, one of the positive electrode elastic sheet (121) and the negative electrode elastic sheet (123) is connected to the abutting valve (110), and the other of the positive electrode elastic sheet (121) and the negative electrode elastic sheet (123) is connected to the infusion cover plate (150).

7. The patch infusion pump of claim 6, wherein the infusion assembly (1) further comprises a guide post (160) provided with limiting guide slots (161) along a circumferential direction of the guide post (160); and
wherein the infusion cover plate (150) and the abutting valve (110) are disposed up and down, the guide post (160) is disposed on a side of the infusion cover plate (150) and the abutting valve (110), and the memory metal wire (122) is disposed around the guide post (160) and is positioned in a respective one of the limiting guide slots (161).

8. The patch infusion pump of claim 6, wherein the infusion assembly (1) further comprises a ground elastic sheet (170), the patch infusion pump further comprises a circuit board (5), the positive electrode elastic sheet (121) is electrically connected to the circuit board (5), and the negative electrode elastic sheet (123) is electrically connected to the circuit board (5) through the ground elastic sheet (170).

9. The patch infusion pump of any one of claims 1 to 8, further comprising: a housing (3), the liquid reservoir (4), a power supply mechanism (6), and the medication output mechanism (10),
wherein a mounting cavity is formed in the housing (3), the infusion assembly (1), the liquid reservoir (4), and the power supply mechanism (6) are all disposed in the mounting cavity, a liquid inlet end of the infusion tube (2) communicates with the liquid reservoir (4), a liquid outlet end of the infusion tube (2) communicates with the medication output mechanism (10), and the power supply mechanism (6) is configured to supply power to the patch infusion pump.

10. An infusion method, being applied in the patch infusion pump of any one of claims 1 to 9, wherein the infusion assembly (1) of the patch infusion pump comprises the infusion tube (2) and the plurality of valve mechanisms (9), and the plurality of valve mechanisms (9) comprise a first valve mechanism (91) located most upstream, a third valve mechanism (93) located most downstream, and a second valve mechanism (92) located between the first valve mechanism (91) and the third valve mechanism (93) along the flow direction of the liquid medication in the infusion tube (2); and the infusion method comprises:
performing, by the infusion assembly (1), one infusion operation according to the following sequence: a memory metal member (120) of the first valve mechanism (91) is energized, a memory metal member (120) of the second valve mechanism (92) is energized, the memory metal member (120) of the first valve mechanism (91) is de-energized, a memory metal member (120) of the third valve mechanism (93) is energized, the memory metal member (120) of the second valve mechanism (92) is de-energized, and the memory metal member (120) of the third valve mechanism (93) is de-energized.
